# EUROPÄISCHE PATENTSCHRIFT

(11) **EP 3 864 310 B1**
(45) Veröffentlichungstag und Bekanntmachung des Hinweises auf die Patenterteilung: **04.12.2024**
(21) Anmeldenummer: 19786945.6
(22) Anmeldetag: 10.10.2019
(51) Int. Cl.: F16C 35/04, F16C 35/067, F16C 25/06, F16C 19/18, F16C 33/58, F16C 19/38, F16C 43/08, A61M 5/145

(54) **DOPPELREIHIGE WÄLZLAGEREINHEIT MIT VORSPANNELEMENT**
DOUBLE-ROW ROLLER BEARING UNIT WITH PRELOAD ELEMENT
PALIER À ROULEAUX À DOUBLE RANGÉE AVEC ELEMENT DE PRE-CHARGE

(30) Priorität: 12.10.2018 DE 102018125316
(43) Veröffentlichungstag der Anmeldung: 18.08.2021
(73) Patentinhaber: B. Braun Melsungen AG, 34212 Melsungen (DE)
(72) Erfinder: STICH, Marcel, 34329 Nieste (DE)
(74) Vertreter: Winter, Brandl - Partnerschaft mbB
(86) Internationale Anmeldenummer: PCT/EP2019/077494
(87) Internationale Veröffentlichungsnummer: WO 2020/074649

(56) Entgegenhaltungen:
- EP-A1- 2 908 902
- EP-B1- 2 908 902
- DE-B3- 102006 034 729
- DE-U1- 8 009 464
- DE-U1- 8 009 464
- US-A1- 2010 284 643

## Beschreibung

Die vorliegende Erfindung betrifft eine doppelreihige Wälzlagereinheit einer medizinischen Pumpe mit einem Lagerkern, welcher eine erste, in eine Axialrichtung gewandte Innenlauffläche für erste Wälzkörper sowie eine zweite, der ersten Innenlauffläche in Axialrichtung entgegengesetzt angeordnete Innenlauffläche für zweite Wälzköper ausbildet, einer Lagerbuchse, welche an einem Gehäuseabschnitt anbringbar ist und eine der ersten Innenlauffläche gegenüberliegende erste Außenlauffläche ausbildet, und einem Lagertopf, welcher eine der zweiten Innenlauffläche gegenüberliegende zweite Außenlauffläche ausbildet. Ferner betrifft die vorliegende Erfindung eine medizinische Pumpe, vorzugsweise Spritzenpumpe, mit einer entsprechenden doppelreihigen Wälzlagereinheit.

### Technischer Hintergrund

Medizintechnische Therapien erfordern heute hochpräzise medizinische Pumpen, welche hohe Anforderungen unter anderem an Bauraum, Gewicht, Bedienungs- und Funktionssicherheit sowie Genauigkeit haben. Entsprechend unterliegen die einzelnen mechanischen Komponenten engen Toleranzen, hohen Miniaturisierungsanforderungen und müssen ggf. aufwändig eingebaut und eingestellt werden.

Beispielsweise werden in der Infusionstherapie zur Applikation von Flüssigkeiten häufig Spritzenpumpen eingesetzt, wie z.B. die Perfusor^{®} Space von B.Braun, welche flexible, sichere Therapien ermöglichen und hochpräzise arbeiten müssen, um auch minimale Flüssigkeitsmengen genau dosieren zu können. In einer solchen Spritzenpumpe wird eine mit einer solchen Flüssigkeit gefüllte Spritze in die Pumpe eingelegt und die Spritze wird anschließend über einen Antriebskopf mit einer definierten Förderrate ausgedrückt (Förderbetrieb), welcher über eine Spindel angetrieben wird. Dabei werden über einen langen Zeitraum, bis zu mehreren Tagen, auf die Spindel hohe Axiallasten bei kleinen Drehzahlen aufgebracht. Muss die Spritze gewechselt werden, wird der Antriebskopf schnell, d.h. in wenigen Sekunden, mit hohen Drehzahlen und sehr geringen Axiallasten zurückgefahren (Rückstellbetrieb). Aufgrund der hohen Genauigkeitsanforderungen werden entsprechend hohe Anforderungen an Toleranzen bei der Lagerung der Spindel gestellt.

Derzeit werden für derartige Präzisionsanwendungen Lagerkonzepte verwendet, welche aus einer Vielzahl einzelner Lager bestehen. Die für die einzelnen Lager notwendigen Bauelemente und -gruppen müssen einzeln eingelagert, kommissioniert und eingebaut werden. Die Lagerungen werden während der Montage der Pumpe aufwändig eingestellt, was zusätzlich dadurch verschärft wird, dass der zur Verfügung stehende Bauraum sehr gering ist und hohe Miniaturisierungsanforderungen vorliegen. Insbesondere ist ein Befestigen der Lagersitze mittels Schrauben aufgrund des beschränkten Bauraums der Pumpe nur eingeschränkt oder gar nicht möglich. Entsprechend ist der Montageaufwand der Pumpe hoch und es sind kostenintensive und fehleranfällige Einstellarbeiten notwendig, was einen reproduzierbar präzisen Einbau der Lagerungen erschwert. Ferner weisen diese Lagerungen ggf. mangelnde Bewegungsfreiheitsgrade auf, wodurch ein Verklemmen der Lagerung insbesondere während des Einbaus derselben auftreten kann. Zudem liegt in den aktuell verwendeten Lagerkonzepten hohe Reibung vor.

Beispielsweise ist aus DE 80 09 464 U1 ein Lager mit einer Tragplatte, einem Außengehäuse und einem Innenring sowie mit zwei axial hintereinander angeordneten Kugelreihen offenbart. Der Innenring umfasst zwei tiefgezogene Blechschalen mit jeweils einer rinnenförmigen Innenlauffläche; und der Außenring umfasst tiefgezogene Blechteile mit rinnenförmigen Außenlaufflächen. Eines der Blechteile weist einen axial nach außen vorspringenden Ringsteg auf, der den Stirnrand des anderen Blechteils klemmend übergreift. DE 80 09 464 U1 offenbart eine doppelreihige Wälzlagereinheit gemäß dem Oberbegriff des Anspruchs 1.

DE 10 2006 034729 B3 betrifft ein zweireihiges Pendelrollen- oder -kugellager, bei dem Wälzkörper zwischen zwei Innenringlaufbahnen und zwei Außenringlaufbahnen angeordnet sind, wobei sowohl die Innenringlaufbahnen als auch die Außenringlaufbahnen durch Bleche gebildet werden, wobei die Innenringlaufbahnen von zwei Stützkörpern aufgenommen werden und wobei die beiden Stützkörper mit Mitteln ausgestattet sind, die ein formschlüssiges Verbinden beider Stützkörper zum unverschieblichen Zusammenhalt in axialer Richtung ermöglichen. Die Mittel sind nach Art eines Reißverschlusses ausgeführt, indem nämlich hakenförmig ausgebildete Vorsprünge und Hinterschnitte in den beiden miteinander zu verbindenden Stützkörpern zusammenwirken.

US 2010/284643 A1 offenbart ein Wälzlager mit einem Lagerinnen- und Lageraußenring, bei dem mindestens einer der Lagerringe in zwei formgleiche Ringhälften geteilt ist. Rastelemente sind an einem radial inneren oder radial äußeren Ringteil angeformt. Segmentartige und schalenförmige Vorsprünge ragen axial über das Ringteil an einem Teil seiner Umfangserstreckung hinaus. Die Vorsprünge sind in einer anderen Radialebene zu den Rastgliedern angeordnet, die in Umfangsrichtung durch segmentartige Vertiefungen begrenzt sind und die Vorsprünge der einen Ringhälfte in die formkomplementär ausgebildeten Vertiefungen der anderen Ringhälfte eingreifen, sodass sich die Vorsprünge und das Ringteil in axialer Richtung überlappen und die Rastglieder der einen Ringhälfte in Aussparungen eingreifen, die im anderen Ringteil angeordnet sind.

Demgemäß besteht die Aufgabe der vorliegenden Erfindung darin, aus dem Stand der Technik bekannte Nachteile zu beheben oder zu reduzieren, und insbesondere darin, eine Montage von medizinischen Pumpen zu vereinfachen, sicherer, präziser und reproduzierbar zu machen und die Fertigungskosten zu senken.

### Zusammenfassung der Erfindung

Die der Erfindung zugrunde liegende Aufgabe wird durch eine doppelreihige Wälzlagereinheit einer medizinischen Pumpe, vorzugsweise Spritzenpumpe, gelöst, mit einem Lagerkern, welcher eine erste, teilweise in eine Axialrichtung gewandte Innenlauffläche für erste Wälzkörper sowie eine zweite, der ersten Innenlauffläche teilweise in Axialrichtung entgegengesetzt angeordnete Innenlauffläche für zweite Wälzköper ausbildet. Ferner hat die Pumpe eine Lagerbuchse, welche an einem Gehäuseabschnitt bzw. einer Lagerplatte anbringbar ist und eine der ersten Innenlauffläche gegenüberliegende erste Außenlauffläche für die ersten Wälzkörper ausbildet, sowie einen Lagertopf oder Lagerdeckel, welcher eine der zweiten Innenlauffläche gegenüberliegende zweite Außenlauffläche für die zweiten Wälzkörper ausbildet. Dabei ist zumindest ein Vorspannelement vorgesehen, welches den Lagertopf mit der Lagerbuchse mit einer definierten Vorspannung/Anstellung koppelt, um die handhabbar vormontierte, doppelreihige Wälzlagereinheit auszubilden. Vorzugsweise ist die vorgespannte Wälzlagereinheit vorspannungseinstellfrei in der Pumpe einbaubar.

An der Wälzlagereinheit sind ferner radial nach außen vorstehende Stützelemente ausgebildet, welche zur Abstützung in einer zweiten Lastrichtung an dem Gehäuseabschnitt vorgesehen sind. Die Stützelemente sind elastisch, um radial nach innen einfedern zu können. Insbesondere ist die erste Lastrichtung eine Nebenlastrichtung, in welcher Kräfte beispielsweise bei einem Rückstellbetrieb der Spritzenpumpe wirken. Durch radial nach innen einfederbare Stützelemente wird dabei der Einbau der Wälzlagereinheit in der Pumpe vereinfacht, da diese dabei aus dem Weg gedrückt werden können. Ferner kann ein Zurückfedern der Stützelemente als ein Signal für ein Erreichen der korrekten Einbauposition dienen.

In anderen Worten wird eine angestellte Lagerung in X- oder O-Anordnung bereitgestellt, welche durch zumindest ein Vorspannelement als vormontierte und vorgespannte, spielfreie Einheit bereitgestellt ist. Eine Lagerung mit X-Anordnung bedeutet im Rahmen der vorliegenden Erfindung, dass zwei kombinierte Schräglagerabschnitte derart spiegelbildlich zueinander angeordnet sind, dass Drucklinien (Richtungen, entlang welcher in einer Reihe von Wälzkörpern Kräfte übertragen werden) sich in der entsprechenden Lagerachse schneiden und dabei in Richtung der jeweils gegenüberliegenden Reihe von Wälzkörpern bzw. dem gegenüberliegenden Schräglagerabschnitt weisen. Bei einer O-Anordnung verlaufen diese Drucklinien in entgegengesetzte Richtung, also in Richtung radial innen weg von dem gegenüberliegenden Schräglagerabschnitt. Eine solche Lagerung ermöglicht eine sehr enge, präzise Führung einer Welle oder Spindel der medizinischen Pumpe. Ferner ist die Anzahl der benötigten Bauteile gegenüber einer konventionellen Lagerung deutlich reduziert, sodass die Kosten reduziert sind. Als Wälzkörper kommen beispielsweise Kegel, Rollen oder Nadeln, sowie vorzugsweise Kugeln, Tonnen und Pendelrollen in Frage. Dabei können die ersten und zweiten Wälzlager abhängig von den auftretenden Belastungen jeweils unterschiedlich geformt und/oder dimensioniert sein. Die Wälzlagereinheit ist vorzugsweise lebensdauergeschmiert.

Vorzugsweise ist die Lagerbuchse über einen Passungsabschnitt an dem Gehäuseabschnitt anbringbar ist, wobei der Lagertopf entlang der Axialrichtung neben dem Passungsabschnitt und diesen nicht überlappend angeordnet ist. In anderen Worten ausgedrückt sind der Passungsabschnitt und der Lagertopf im zusammengebauten Zustand der Lagereinheit vorzugsweise in Axialrichtung zueinander versetzt bzw. zueinander beabstandet angeordnet. Weiter vorzugsweise ist das zumindest eine Vorspannelement ein elastischer, die Lagerbuchse zumindest teilweise umgreifender Abschnitt des Lagertopfs. In anderen Worten ausgedrückt sind der Lagertopf und das Vorspannelement stoffeinstückig ausgebildet. Insbesondere ist der Lagertopf an seinem der Lagerbuchse zugewandten Ende geschlitzt und zwischen den Schlitzen stehenbleibende Stege sind elastisch biegbar, um mehrere Vorspannelemente zu bilden. Vorteile dieser Ausgestaltung liegen darin, dass ein radialer Bauraum der Lagereinheit verringert werden kann und/oder die Montage derselben vereinfacht ist und/oder die Bearbeitung des Lagersitzes einfacher ist und/oder das zumindest eine Vorspannelement hinsichtlich seiner Biegbarkeit und Handhabbarkeit vorteilhaft ausgebildet werden kann.

Eine erfindungsgemäße Wälzlagereinheit ermöglicht, den Aufwand deren Einbaus in die Pumpe sowie der zugehörigen Vorbereitung deutlich zu reduzieren. Anstelle mehrerer verschiedener Lager kann die Wälzlagereinheit als einzelne, vormontierte Baugruppe transportiert, gelagert und kommissioniert werden und anschließend in die Pumpe eingebaut werden, ohne dass ein Arbeitsschritt zur Einstellung der Vorspannung der Lagerung vorgesehen werden muss. Beispielsweise kann die Wälzlagereinheit auf eine Welle oder Spindel einer Pumpe aufgepresst werden und über eine entweder an dem Lagertopf oder an der Lagerbuchse vorgesehenen Passungsabschnitt an dem Pumpengehäuse befestigt werden. Alternativ kann die Wälzlagereinheit beispielsweise in das Pumpengehäuse eingeklebt, eingepresst oder eingeschraubt werden. Hierfür kann z.B. an einem Außenumfang der Wälzlagereinheit ein Gewinde bereitgestellt sein.

Es sind zudem verschiedene Ausführungen des zumindest einen Vorspannelements denkbar. Es kann als separates, elastisches Bauteil bereitgestellt werden, wie z.B. als eine in die Wälzlagereinheit eingelegte Zug- oder Druckfeder, oder auch als eine Gruppe von separaten, elastischen Bauteilen, die vorzugsweise in gleichen Abständen rund um den Umfang der Wälzlagereinheit verteilt angeordnet sind. Denkbar ist auch, ein an dem Lagertopf und/oder der Lagerbuchse vorgesehenes Gewindeelement als das Vorspannelement bereitzustellen und somit den Lagertopf und die Lagerbuchse durch ein Schraubgewinde, insbesondere ein Einstellgewinde, miteinander zu verspannen.

Es hat sich als besonders zweckmäßig erwiesen, dass das zumindest eine Vorspannelement zumindest ein elastischer Abschnitt des Lagertopfs oder der Lagerbuchse ist, vorzugsweise eine Anzahl von Schnapphaken. Weiter vorzugsweise ist eine Anzahl von Vorspannelementen in gleichen Abständen rund um den Umfang der Lagerbuchse oder des Lagertopfes verteilt. Dieses zumindest eine Vorspannelement umgreift entsprechend die Lagerbuchse bzw. den Lagertopf zumindest teilweise oder greift darin ein. Somit ist das zumindest eine Vorspannelement mit anderen Bauteilen der Wälzlagereinheit integral verbunden, sodass die Anzahl der benötigten Bauteile reduziert und das Gewicht der Wälzlagereinheit optimiert werden kann, sowie Fertigungskosten gesenkt werden können. Insbesondere ermöglicht die Ausgestaltung des Vorspannelements als ein ein-/umgreifendes elastisches Bauteil wie z.B Schnapphaken, dass die Vorspannung der Wälzlagereinheit durch diese Elastizität und eine konstruktive Ausgestaltung festgelegt ist. Besonders aufgrund der durch den beschränkten Bauraum bedingten Miniaturisierung der Wälzlagereinheit ist dies eine erhebliche Erleichterung. Beispielsweise gegenüber einer Einstellung durch ein Einstellgewinde hat dies zum Vorteil, dass die Vormontage der Wälzlagereinheit deutlich schneller erfolgt und die hohen Anforderungen an Toleranzen zuverlässig eingehalten werden, da der Prozess besser reproduzierbar ist, d. h., weniger menschlichen Fehlern unterliegt. Dadurch werden die Fertigungskosten erheblich gesenkt.

Eine vorteilhafte Ausgestaltung der erfindungsgemäßen Wälzlagereinheit sieht vor, dass die ersten Wälzkörper und ersten Laufflächen mit den zweiten Wälzkörpern und zweiten Laufflächen eine X-angeordnete Lagerung bilden. Insbesondere wird bevorzugt, dass die ersten und/oder zweiten Wälzkörper eine Taumelbewegung des Lagerkerns relativ zu dem Lagertopf und der Lagerbuchse ermöglichen (Taumelfreiheit), vorzugsweise in einem Winkelbereich von +/-1° um die Lager- bzw. Längsachse.

Eine Taumelfreiheit in der Wälzlagereinheit ist wichtig für einen möglichst schnellen, reproduzierbaren und sicheren Einbau der erfindungsgemäßen Wälzlagereinheit, da die Welle oder Spindel der medizinischen Pumpe in der Regel nicht perfekt koaxial zu der Lagerachse und/oder zu einem an dem Gehäuseabschnitt bereitgestellten Lagersitz eingesetzt werden kann. Bei dem Einbau, insbesondere bei einer Handhabung der Spindel oder Welle kommt es entsprechend zu geringfügigen Winkelabweichungen, welche durch die Wälzlagereinheit zugelassen werden müssen. Eine X-Anordnung der ersten und zweiten Wälzlagerreihen hat ein relativ großes Kippspiel, d. h., sie ist dazu geeignet, geringfügige Winkelabweichungen zwischen dem Lagerkern sowie der Lagerbuchse und dem Lagertopf zuzulassen, ohne sich zu verklemmen. Ferner werden nach dieser bevorzugten Ausführung der Erfindung abgerundete Wälzkörper, d. h., Tonnen, Pendelrollen oder vorzugsweise Kugeln eingesetzt, damit das Taumeln möglich ist.

Sinnvollerweise kann die Lagerbuchse einen radial nach außen vorstehenden Flansch ausbilden, welcher zur Abstützung in einer ersten Lastrichtung an dem Gehäuseabschnitt vorgesehen ist.

Da die Wälzlagereinheit derart eingebaut wird, dass der Flansch an dem Gehäuseabschnitt anliegt, wird durch diesen die axiale Einbauposition der Wälzlagereinheit festgelegt. Dies ist für den präzisen und reproduzierbaren Einbau der Wälzlagereinheit in der medizinischen Pumpe wichtig. Zudem dient der Flansch als Abstützung der von der Wälzlagereinheit auf den Gehäuseabschnitt übertragenen axialen Kräfte in der ersten Lastrichtung, welche vorzugsweise einer Hauptlastrichtung entspricht, beispielsweise bei einem Förderbetrieb der medizinischen Spritzenpumpe. Wenn sowohl der Flansch als auch die Stützelemente vorgesehen sind, ist die Wälzlagereinheit axial in beide Richtungen formschlüssig gesichert.

Die erfindungsgemäße Wälzlagereinheit ist derart ausgebildet, dass das zumindest eine Vorspannelement einen oder mehrere Niederhalter aufweist, welche dazu konfiguriert sind, sich radial nach außen an dem Gehäuseabschnitt abzustützen, um das zumindest eine Vorspannelement in eine zugehörige Eingriffskontur zu drücken, wenn das doppelreihige Wälzlager in dem Gehäuseabschnitt eingebaut ist. Dies ist insbesondere vorteilhaft, wenn als Vorspannelemente Schnapphaken vorgesehen sind, welche mit einem radial nach innen abstehenden Vorsprung zumindest teilweise die Lagerbuchse bzw. den Lagertopf umgreifen oder darin eingreifen, wobei die Schnapphaken bei der (Vor-) Montage und Demontage der Wälzlagereinheit radial nach außen aufgebogen werden müssen. Die Niederhalter verhindern das Aufbiegen nach außen der Schnapphaken, wenn die Wälzlagereinheit in der Pumpe eingebaut ist, und dienen somit als eine Selbstsicherung gegen ein Öffnen der Lagereinheit im Betrieb. Dies ist insbesondere dann vorteilhaft, wenn die medizinische Pumpe eine Spritzenpumpe mit einer Zugspindel ist, da bei einer solchen bei einem Förderbetrieb hohe Zugkräfte auf die Wälzlagereinheit aufgebracht werden, durch welche sich die Schnappverbindung ggf. ungewollt öffnen könnte.

Alternativ ist es denkbar, einen Niederhalter, insbesondere in Form einer umlaufenden, nach radial innen vorstehenden Auskragung oder Stufe, an dem Gehäuseabschnitt vorzugsehen, welcher von radial außen gegen die Vorspannelemente oder Schnapphaken drückt. Je nachdem, wie der Gehäuseabschnitt strukturell ausgestaltet ist und welchen Anforderungen er ferner unterliegt, ist diese Lösung kostengünstiger als die, bei welcher die Niederhalter von den Vorspannelementen ausgebildet sind.

Zusammenfassend ist anzumerken, dass die erfindungsgemäße Wälzlagereinheit in Ihrer einfachsten Form Vorspannelemente vorsieht. Die Stützabschnitte sowie die Niederhalter sind vorteilhafte Ausgestaltungen der Erfindung, welche sowohl einzeln als auch kombiniert und ggf. einstückig ausgebildet vorgesehen werden können, um vorstehend beschriebene, vorteilhafte Effekte zu erzielen.

Die Wälzlagereinheit ist spielfrei und kompakt, ihr Einbau in die medizinische Pumpe ist sehr einfach und reproduzierbar, da keine Einstellarbeiten oder Prüfungen der Vorspannung notwendig sind, und die Stückkosten sind gering, da die einzelnen Komponenten der Wälzlagereinheit günstig durch Volumenprozesse fertigbar sind, wie z.B. durch Spritzgießen aus Tribo-Kunststoff. Ebenso ist die (Vor-)Montage der Wälzlagereinheit an sich, insbesondere in der Ausführung mit Schnapphaken, schnell, kostengünstig und reproduzierbar, da die Komponenten, insbesondere die Schnapphaken, selbsteinstellend sind.

Die Wälzlagereinheit wird vormontiert, indem in die Lagerbuchse oder das Lagergehäuse die Wälzkörper, insbesondere Kugeln, und der Lagerkern eingelegt werden, welche anschließend durch ein Anbringen, insbesondere Aufschnappen, des Lagertopfs oder Lagerdeckels definiert spielfrei verspannt werden. Dabei drückt der Lagertopf gegen die Kugeln, welche einen Anschlag bzw. eine Begrenzung des Vorspannwegs ausbilden. Ferner werden die Kugeln bei dem Aufschnappen des Lagertopfs in ihre für den Betrieb vorgesehenen Positionen gedrückt bzw. kommen in ihren vorbestimmten Laufflächen/Laufbahnen zu liegen. Dadurch wird insbesondere ein X-spielfrei verspanntes Lagerelement ausgebildet. Das heißt, die Vorspannung wird durch die Montage der Wälzlagereinheit an sich erreicht. Der Lagertopf, insbesondere in der Ausführung mit Schnapphaken, ist durch seine Formgebung bedingt federnd und lässt sich bei der Montage in eine Rastgeometrie einhaken. Dadurch wird eine dauerhafte (Vorspann-)Kraft auf den drehbaren Innenläufer/Lagerkern aufgebracht. Sowohl die Montage der Wälzlagereinheit an sich als auch deren Einbau in der Pumpe sind leicht automatisierbar.

Des Weiteren wird die der Erfindung zugrunde liegende Aufgabe durch eine medizinische Pumpe, vorzugsweise eine Spritzenpumpe, gelöst, welche eine Spindel, vorzugsweise eine Zugspindel, aufweist, die durch eine doppelreihige Wälzlagereinheit gemäß der vorstehenden Beschreibung drehbar und axialfest in einem Gehäuseabschnitt der medizinischen Spritzenpumpe gelagert ist.

Die vormontierte Wälzlagereinheit kann in der Pumpe mittels eines ggf. miniaturisierten Einpresswerkzeugs eingebaut werden. Dabei wird sie in eine beliebige Bohrung mit einer entsprechenden Passung eingepresst. Die Festigkeit der Verbindung wird durch die Passung zwischen der Bohrung und der Lagereinheit und durch die Scherfestigkeit eines Lagerbundes (Passungsabschnitts) definiert.

### Figurenbeschreibung

Nachfolgend wird die vorliegende Erfindung anhand beispielhafter Ausführungsformen mit Referenz auf die beigefügten Zeichnungen beschrieben, die Erfindung soll jedoch nicht darauf begrenzt sein. Dabei werden für gleiche Elemente dieselben Bezugszeichen verwendet.
Fig. 1 zeigt eine Spritzenpumpe, in welcher eine doppelreihige Wälzlagereinheit eingebaut ist.
Fig. 2 zeigt eine perspektivische Ansicht der doppelreihigen Wälzlagereinheit nach einem Beispiel zum Verständnis der Erfindung.
Fig. 3 zeigt eine Längsschnittansicht der doppelreihigen Wälzlagereinheit nach dem Beispiel nach Fig. 2.
Fig. 4 zeigt eine perspektivische Ansicht der doppelreihigen Wälzlagereinheit nach einer Ausführungsform der Erfindung.
Fig. 5 zeigt eine Längsschnittansicht der doppelreihigen Wälzlagereinheit nach der Ausführungsform der Erfindung.

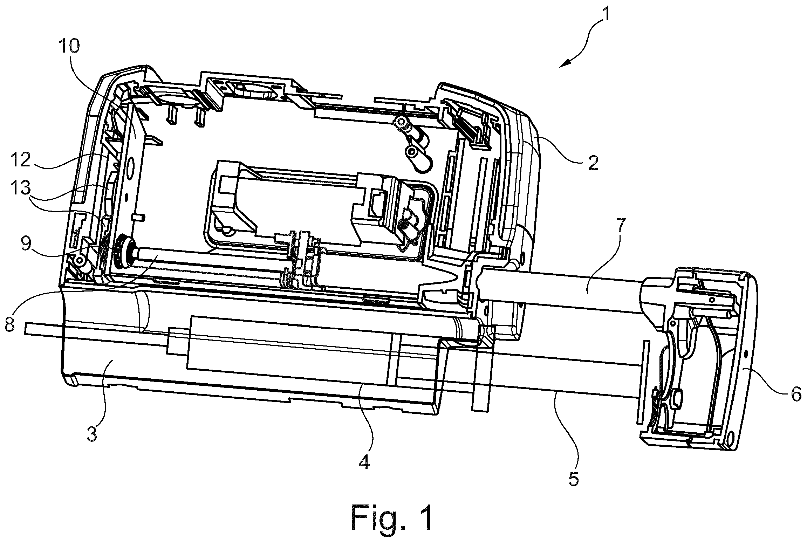

Bei der in Fig. 1 dargestellten medizinischen Pumpe handelt es sich um eine Spritzenpumpe 1, welche beispielsweise bei einer Infusionstherapie zum Einsatz kommt. Die Spritzenpumpe 1 weist ein Spritzengehäuse 2 mit einem Aufnahmefach 3 auf, welches dazu dient, eine Spritze 4 (hier schemenhaft angedeutet) aufzunehmen und gegebenenfalls zu halten. Bei Betrieb der Spritzenpumpe 1 ist eine mit Medikamenten o.Ä. gefüllte Spritze 4 mit einem Spritzenkolben 5 zum Ausdrücken der Spritze 4 in das Aufnahmefach 3 eingelegt und liegt an einem Antriebskopf 6 der Spritzenpumpe 1 an. Der Antriebskopf 6 ist axial verschiebbar, um den Spritzenkolben 5 zu betätigen. Dabei ist er über ein axial verschiebbar an dem Spritzengehäuse 2 gelagertes Antriebsrohr 7 mit einer Spindel 8, genauer einer Zugspindel, verbunden. Zwischen dem Antriebsrohr 7 und der Spindel 8 ist ein Wandlungsmechanismus (nicht dargestellt) vorgesehen, welcher eine Drehbewegung der Spindel 8 in eine Längsbewegung des Antriebskopfes 6 wandelt.

Nachfolgend werden zur besseren Orientierung die Seite der Spritzenpumpe 1 und der Wälzlagereinheit 9, welche in Richtung des Antriebskopfes 6 bzw. in eine Zugrichtung betrachtet gewandt sind, als Vorderseite bezeichnet. Die dazu entgegengesetzte Seite bzw. Richtung der Spritzenpumpe 1 und der Wälzlagereinheit 9, welche in Richtung des Getriebeabschnitts 12 bzw. in eine Druckrichtung gewandt ist, wird als Rückseite bezeichnet.

Die Spindel 8 ist durch eine doppelreihige Wälzlagereinheit 9 drehbar und axialfest an dem Spritzengehäuse 2 gelagert. Nach dieser Ausführungsform sitzt die doppelreihige Wälzlagereinheit 9 in einem von einer Lagerplatte 10 ausgebildeten Lagersitz 11. Die Lagerplatte 10 ist ein Gehäuseabschnitt, welcher dazu dient, in dem Spritzengehäuse 2 einen Getriebeabschnitt 12 abzuteilen, in welchem Antriebszahnräder 13 zum Antreiben der Spindel 8 angeordnet sind. Die Antriebszahnräder 13 sind ebenfalls an der Lagerplatte 10 gelagert. Zum Antreiben der Antriebszahnräder 13 ist ferner ein Elektromotor (nicht dargestellt) vorgesehen.

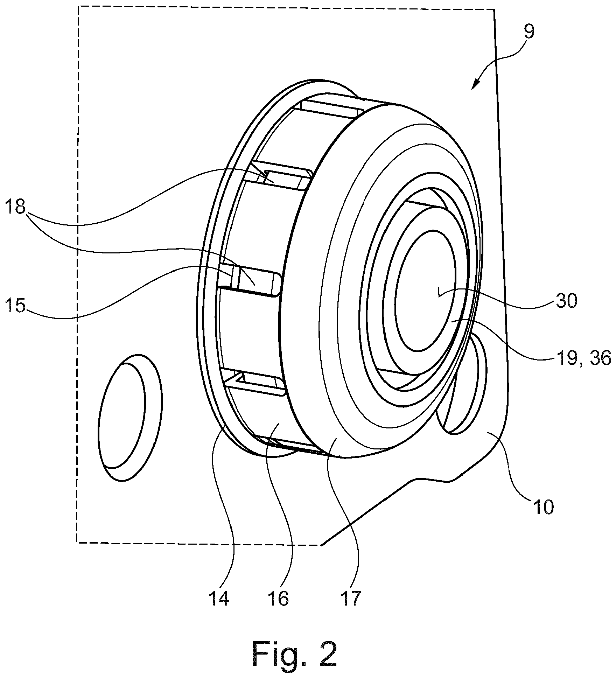

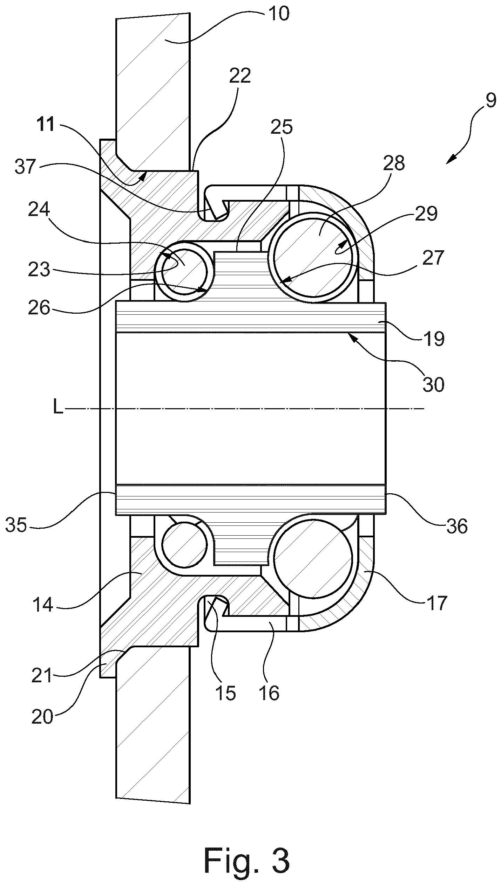

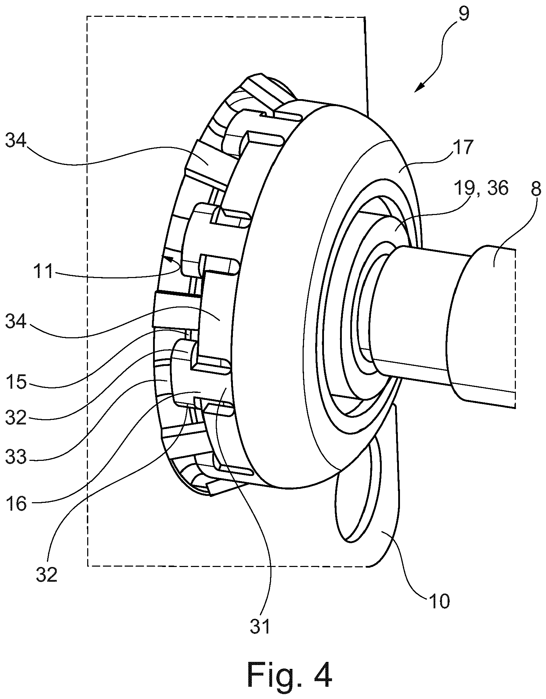

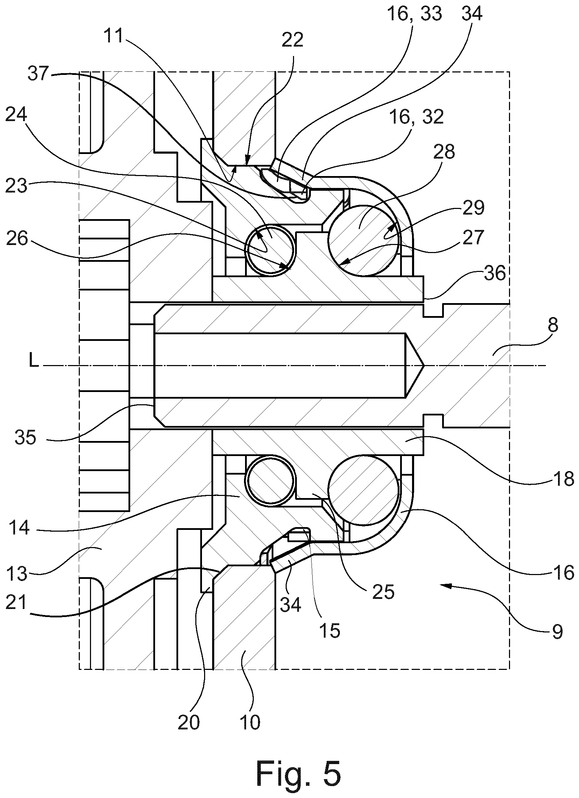

Das nachfolgend mit Verweis auf Fig. 2 und Fig. 3 beschriebene Beispiel dient lediglich zum Verständnis der anschließend mit Verweis auf Fig. 4 und 5 beschriebenen Ausführungsform der vorliegenden Erfindung.

Fig. 2 ist eine perspektivische Darstellung der doppelreihigen Wälzlagereinheit 9. Ein Lageraußenring der Wälzlagereinheit 9 ist zweiteilig und weist eine Lagerbuchse 14 und einen Lagertopf 17 auf. Die Lagerbuchse 14 bildet außenseitig eine Eingriffskontur in Form einer Umfangsnut 15 aus, in welche Schnapphaken 16 des Lagertopfs 17 eingreifen. Die Schnapphaken 16 dienen dabei als ein Vorspannelement, durch welches eine definierte Vorspannung/Anstellung der Wälzlagereinheit 9 erzeugt wird. Die Schnapphaken 16 sind dadurch ausgebildet, dass der Lagertopf 17 an seinem rückseitigen (d. h., im eingebauten Zustand der Lagerplatte 10 des Spritzengehäuses 2 zugewandten), hülsenförmig ausgebildeten Ende in gleichen Abständen rund um den Lagerumfang verteilte Schlitze 18 aufweist, welche in Axialrichtung verlaufen. Zwischen den Schlitzen 18 bleiben elastische Stege, die Schnapphaken 16, stehen. An seinem vorderseitigen (d. h., im eingebauten Zustand der Lagerplatte 10 des Spritzengehäuses abgewandten) Ende ist der Lagertopf 17 schüsselartig nach radial innen gekrümmt und in Axialrichtung offen. Innerhalb der Lagerbuchse 14 und des Lagertopfs 17 ist ein Lagerkern 19 angeordnet, dessen vorderseitiges Ende, wie in Fig. 2 dargestellt, radial innerhalb einer Öffnung des der Lagerplatte 10 abgewandten Endes des Lagertopfs 17 liegt. Die Wälzlagereinheit 9 ist als eine vormontierte Baugruppe an der Lagerplatte 10 montiert, indem die Lagerbuchse 14 in die Lagerplatte 10 eingepresst ist.

Fig. 3 zeigt einen Längsschnitt der Wälzlagereinheit 9 des in Fig. 2 beschriebenen Beispiels. Eine Position der Wälzlagereinheit 9 in Axialrichtung ist dadurch festgelegt, dass ein von der Lagerbuchse 14 ausgebildeter, sich radial nach außen erstreckender Kragen oder Flansch 20 rückseitig (d. h., an der Seite des Getriebeabschnitts 12 bzw. an der dem Antriebskopf 6 abgewandten Seite) an der Lagerplatte 10 anliegt. Ferner wird durch den Flansch 20 eine während eines Förderbetriebs auf die Spindel 8 wirkende Zugbelastung (d. h., eine in Zugrichtung, in Richtung hin zu dem Antriebskopf 6 wirkende Hauptlast) an der Lagerplatte 10 abgestützt. Rund um den Lagersitz 11 an der Rückseite der Lagerplatte 10 ist eine Fase 21 vorgesehen, um ein Einpressen bzw. einen Einbau der Lagerbuchse 14 an der Leiterplatte 10 zu erleichtern. Unmittelbar vorderseitig des Flanschs 20 weist die Lagerbuchse 14 einen Passungsabschnitt 22 auf, welcher sich in Axialrichtung erstreckt und über welchen die Lagerbuchse 14 wie vorstehend beschrieben in den durch die Lagerplatte 10 ausgebildeten Lagersitz 11 eingepresst ist. Durch die so erzeugte Pressverbindung wird eine auf die Spindel 8 wirkende Druckbelastung (d.h., in Druckrichtung bzw. in Richtung der Rückseite wirkende Nebenlast) auf die Lagerplatte 10 übertragen. Unmittelbar hinter dem Passungsabschnitt 22 bildet die Lagerbuchse 14 in der folgenden Reihenfolge eine radial nach innen verlaufende Stufe, die Umfangsnut 15 und einen sich in Axialrichtung zu der Vorderseite hin erstreckenden Endabschnitt aus.

An einer Innenseite der Lagerbuchse 14, genauer, an einem radial innerhalb des Passungsabschnitts 22 und des Lagersitzes 11 liegenden Abschnitt der Lagerbuchse 14, ist ein sich radial nach innen erstreckender Bund vorgesehen, welcher an dessen Vorderseite eine erste Außenlauffläche 23 nach Bauart eines Schrägrillenkugellagers ausbildet. Die erste Außenlauffläche 23 stützt erste Wälzkörper 24, in diesem Beispiel Kugeln, rückseitig sowie radial außen.

Innerhalb der Lagerbuchse 14 ist der Lagerkern 19 angeordnet, welcher im Wesentlichen hülsenförmig ist und an einer Innenumfangsfläche einen Spindelsitz 30 für eine Verbindung mit der Spindel 8 ausbildet. Der Spindelsitz 30 ist bevorzugt eine leichte Spiel- oder Übergangspassung, kann jedoch auch als Presspassung ausgebildet sein. In einem mittleren Bereich weist der Lagerkern 19 ferner eine radial nach außen hervorstehende Auskragung 25 auf. An einem Übergang zwischen der Auskragung 25 und rückseitigen Ende des Lagerkerns 19 ist eine erste Innenlauffläche 26 nach Bauart eines Schrägrillenkugellagers für die ersten Wälzkörper 24 ausgebildet, welche der ersten Außenlauffläche 23 derart wälzkörperdiametral gegenüberliegt, dass die ersten Wälzkörper 24 sich bei einer Relativdrehung das Lagerkerns 19 und der Lagerbuchse 14 zwischen der ersten Innenlauffläche 26 und der ersten Außenlauffläche 23 abwälzen.

An einem Übergang der Auskragung 25 und dem vorderseitigen Ende des Lagerkerns 19 ist eine zweite Innenlauffläche 27 nach Bauart eines Schrägrillenkugellagers ausgebildet, um zweite Wälzkörper 28, in diesem Beispiel Kugeln, rückseitig sowie radial innen abzustützen. Der zweiten Innenlauffläche 27 wälzkörperdiametral gegenüberliegend ist an einer Innenseite des Lagertopfs 17, genauer innerhalb von dessen schüsselartig ausgebildetem Ende, eine zweite Außenlauffläche 29 nach Bauart eines Schrägrillenkugellagers ausgebildet, um die zweiten Wälzkörper 28 vorderseitigen sowie radial außen zu stützen. D. h., die zweite Innen- und die zweite Außenlauffläche 27, 29 liegen einander derart gegenüber, dass die zweiten Wälzkörper 28 sich bei einer Relativdrehung des Lagerkerns 19 und des Lagertopfs 17 zwischen der zweiten Innenlauffläche 27 und der zweiten Außenlauffläche 29 abwälzen.

Darüber hinaus ist in Fig. 3 gut zu sehen, dass die durch den Lagertopf 17 ausgebildeten Schnapphaken 16 in die Umfangsnut 15 der Lagerbuchse 14 eingreifen, um dadurch die Wälzlagereinheit 9 zusammenzuhalten. Darüber hinaus ist erkennbar, dass die freien Enden der Schnapphaken 16, welche in die Umfangsnut 15 eingreifen, radial nach innen gebogen sind und an ihrem rückseitigen Ende bzw. ihrer rückseitigen Stirnfläche eine Rampe oder Montageschräge 37 bilden, durch welche die Schnapphaken 16 während eines Montagevorgangs an der Lagerbuchse 14 zur Anlage kommen und nach außen gedrückt bzw. aufgebogen werden. Genauer werden während einer Montage der Wälzlagereinheit 9 der Lagerkern 19 und die Wälzkörper 24, 28 in die Lagerbuchse 14 eingesetzt und wird der Lagertopf 17 von der Vorderseite aus über die Lagerbuchse 14 geschoben, wobei die Schnapphaken 16 auf das vorderseitige Ende der Lagerbuchse 14 treffen und durch die stirnseitigen Montageschrägen 37 elastisch nach außen gebogen werden, bis die Schnapphaken 16 schließlich in die Umfangsnut 15 der Lagerbuchse 14 einschnappen. Dabei können sich die Schnapphaken 16 nicht vollständig elastisch zurückstellen, sondern pressen gegen eine vorderseitige Wand der Umfangsnut 15, wodurch eine definierte Vorspannung bzw. Anstellung der Wälzlagereinheit 9 erreicht wird.

Ferner ist in Fig. 3 zu sehen, dass die unmittelbar hinter dem Passungsabschnitt 22 ausgebildete Stufe der Lagerbuchse 14 radial nach außen über den Außenumfang des Lagertopfs 17 hinausreicht und dadurch die Schnapphaken 16 in Axialrichtung abschirmt. Dadurch wird verhindert, dass insbesondere in einem vormontierten, uneingebauten Zustand der Wälzlagereinheit 9 ein Störkörper hinter die Schnapphaken 16 greift und diese ungewollt geöffnet werden, wodurch die Wälzlagereinheit 9 auseinanderfallen würde.

Bei einer auf die Spindel 8 wirkenden Zugbelastung, d. h., in einem Förderbetrieb der Spritzenpumpe 1, wird eine entsprechende Zugkraft über den Spindelsitz 30 (im Falle der Presspassung des Spindelsitzes 30) oder über die rückseitige Stirnfläche 35 (in Fig. 3 links) des Lagerkerns 19 (im Falle der Spiel- oder Übergangspassung des Spindelsitzes 30) auf den Lagerkern 19 übertragen. Hierfür ist es von Vorteil, wenn der Lagerkern 19 aus einer zugehörigen Öffnung der Lagerbuchse 14 hervorragt. Von dem Lagerkern 19 wird die Zugkraft über die zweite Innenlauffläche 27 des Lagerkerns 19 auf die zweiten Wälzkörper 28 und von dort auf die zweite Außenlauffläche 29 des Lagertopfs 17 übertragen. Die somit auf den Lagertopf 17 wirkende Zugbelastung wird über die Schnapphaken 16 auf die Lagerbuchse 14 und von dort aus über die Reibung in der Pressverbindung zwischen der Lagerbuchse 14 und der Lagerplatte 10 sowie über den Flansch 20 auf die Lagerplatte 10 übertragen. Der Flansch 20 wird benötigt, da bei dem Förderbetrieb relativ hohe Kräfte auf die Spindel 8 wirken. Bei entgegengesetzter Betriebsrichtung, d. h. bei einem Rückstellbetrieb der Spritzenpumpe 1, wirkt eine vergleichsweise geringe Drucklast auf die Spindel 8, welche über den Spindelsitz 30 oder ggf. über eine vorderseitige Stirnfläche 36 (in Fig. 3 rechts) des Lagerkerns 19 auf den Lagerkern 19 übertragen wird. Von dort aus wird die Drucklast über die erste Innenlauffläche 26 und die ersten Wälzkörper 24 auf die erste Außenlauffläche 23 der Lagerbuchse 14 und von dort aus über die Reibung in der Pressverbindung zwischen der Lagerplatte 10 und der Lagerbuchse 14 auf die Lagerplatte 10 übertragen. Gemäß der unterschiedlich großen Belastungen in der Zug- und der Druckrichtung sind nach diesem Beispiel die ersten und zweiten Wälzkörper 24, 28 ausgelegt, sodass als erste Wälzkörper 24 kleinere Kugeln gewählt sind (z.B. mit einem Durchmesser von 2 mm) als für die zweiten Wälzkörper 28 (z.B. mit einem Durchmesser von 3 mm).

Fig. 4 zeigt eine perspektivische Ansicht einer Wälzlagereinheit 9 nach einer Ausführungsform der Erfindung. Der Aufbau der Spritzenpumpe 1, die Wälzlagereinheit 9 an sich sowie deren Einbausituation entsprechen in großen Teilen der des vorstehenden Beispiels, weshalb nachfolgend lediglich Unterschiede zwischen der Ausführungsform und dem vorstehenden Beispiel erläutert werden, um redundante Beschreibungen zu vermeiden.

Bei einem Vergleich von Fig. 4 mit Fig. 2 wird deutlich, dass der größte Unterschied zwischen der Ausführungsform und dem Beispiel in der Ausgestaltung des Lagertopfs 17 liegt, genauer in der Ausgestaltung des sich in Axialrichtung erstreckenden hülsenförmigen Abschnitts des Lagertopfs 17, welcher die Vorspannelemente oder Schnapphaken 16 ausbildet. Die Schnapphaken 16 sind in gleichmäßigen Abständen rund um den Umfang des Lagertopfs 17 verteilt angeordnet. Die Schnapphaken 16 sind nach dieser in Fig. 4 dargestellten Ausführungsform aus im Wesentlichen kreuzförmigen Abschnitten aus Blech, Plastik oder anderen elastisch verformbaren Materialien (insbesondere aus dem gleichen Material und einstückig mit dem übrigen Lagertopf) geformt. Der Fuß 31 dieses Kreuzes ist integral mit dem schüsselförmigen Ende des Lagertopfs 17 verbunden und erstreckt sich axial in Richtung hin zu der Lagerplatte 10 bzw. in Druckrichtung. Zwei Arme 32 des Kreuzes erstrecken sich beiderseitig des Fußes 31 in Umfangsrichtung oder senkrecht dazu und sind mit ihren freien Enden radial nach innen gebogen, um in die Umfangsnut 15 einzugreifen. Dadurch wird die Wälzlagereinheit 9 als Einheit verbunden und wird die Vorspannung der Wälzlagereinheit 9 bereitgestellt. Ein Kopf 33 des Kreuzes stellt im Grunde eine über die Arme 32 hinausgehende Verlängerung des Fußes 31 dar und ist radial nach außen gebogen. Das freie Ende des Kopfes 33 liegt dabei an einer Innenumfangsfläche des in der Lagerplatte 10 ausgebildeten Lagersitzes 11 an, wodurch die kreuzförmigen Schnapphaken 16 im eingebauten Zustand elastisch nach radial innen gedrückt werden oder zumindest in der in die Umfangsnut 15 eingreifenden Lage gehalten werden. D. h., der Kopf 33 des Kreuzes dient als ein Niederhalter zur Sicherung des Eingriffs der Arme 32 des Kreuzes in der Umfangsnut 15, wodurch deren Ausschnappen auch bei einer hohen Zugbelastung verhindert werden kann.

Darüber hinaus bildet der sich in Axialrichtung erstreckende hülsenförmige Abschnitt des Lagertopfs 17 elastische Stützelemente 34 aus. Diese sind rund um den Umfang des Lagertopfs 17 im Wechsel mit den Schnapphaken 16 in gleichen Abständen zueinander angeordnet. Die Stützelemente 34 sind derart schräg nach radial außen abgeknickt, dass diese sich außen an der vorwärts gerichteten Fläche der Lagerplatte 10 abstützen, um zum einen eine Selbstsicherung der Wälzlagereinheit 9 in der Lagerplatte 10 zu realisieren und zum anderen, um auch in Druckrichtung formschlüssig Kräfte auf die Lagerplatte 10 zu übertragen, anstelle dies, wie in dem Beispiel nach Fig. 2 und Fig. 3, ausschließlich reibschlüssig über die Pressverbindung mit dem Lagersitz 11 umzusetzen.

Darüber hinaus sind die Stützelemente 34 über einen verbreiterten, sich parallel zu der Lagerachse erstreckenden Sockel integral mit dem schüsselförmigen Ende des Lagertopfs 17 verbunden und haben ferner ein an den Sockel angeschlossenes, schmaleres freies Ende, welches wie vorstehend beschrieben nach radial außen abgeknickt ist, um sich an der Lagerplatte 10 abzustützen. Dabei liegt fertigungsbedingt der Sockel eines Stützelements 34 zwischen den Füßen 31 zweier kreuzförmiger Schnapphaken 16 und endet der Sockel in Axialrichtung knapp vor zwei Armen 32 zweier Schnapphaken 16. Beispielsweise wird hierdurch eine einfache, gleichmäßige Schnittweite ermöglicht, um die Schnapphaken 16 und die Stützelemente 34 in den einstückig gefertigten Lagertopf 17 einzubringen. Es ist anzumerken, dass in dieser speziellen Ausführungsform sowohl die Schnapphaken 16 als auch die Stützelemente 34 vorgesehen sind.

Fig. 5 zeigt einen Längsschnitt durch die Wälzlagereinheit 9 der Ausführungsform entlang einer Ebene, welche durch die Lagerachse L verläuft und einander bezüglich der Wälzlagereinheit 9 diametral gegenüberliegende Stützelemente 34 schneidet. In dieser Ansicht ist gut erkennbar, dass die Lagerplatte 10 an dem nach vorne gerichteten Umfangsrand des Lagersitzes 11 leicht angeschrägt ist, um eine bessere Auflage der freien Enden der Stützelemente 34 an der Lagerplatte 10 zu ermöglichen. Dadurch, dass die Stützelemente 34 elastisch sind, können diese einfedern, um zu ermöglichen, dass das Lager von der Seite des Getriebeabschnitts 12 ausgehend in den Lagersitz 11 der Lagerplatte 10 eingepasst wird. In Endlage bzw. bei Erreichen des eingebauten Zustands verlassen die Stützelemente 34 den Lagersitz 11 und schnappen nach außen, um sich an der Lagerplatte 10 axial abzustützen.

Ferner ist hinter einem der Stützabschnitte 34 (in der Fig. 5 oben liegend) ein Schnapphaken 16 erkennbar, dessen Kopf 33 sich in radialer Richtung gegen die Innenumfangsfläche des Lagersitzes 11 der Lagerplatte 10 stützt. Gut zu sehen ist außerdem, dass der Passungsabschnitt 22 der Lagerbuchse 14 verglichen mit dem des Beispiels nach Fig. 2 und Fig. 3 relativ kurz ist und nicht in Axialrichtung über den Lagersitz 11 der Lagerplatte 10 hinausreicht, sodass ein Abschnitt des Lagersitzes 11 frei liegt, an welchen sich die Köpfe 33 der Schnapphaken 16 abstützen können. Ferner ist ein Arm 32 des Schnapphakens 16 erkennbar, welcher in die Umfangsnut 15 der Lagerbuchse 14 eingreift und zudem an seiner rückseitigen Stirnfläche eine Rampe oder Montageschräge 37 ausbildet, durch welche der Schnapphaken 16 beim Aufschnappen des Lagertopfs 17 auf die Lagerbuchse 14 nach radial außen gedrückt wird.

In anderen Worten ist in der Ausführungsform die Geometrie des Lagertopfs 9 derart erweitert, dass nach dem Einpressen im Lagersitz 11 Haltelaschen bzw. Stützelemente 34 einrasten und das Lager auch in Druckrichtung formschlüssig und nicht nur reibschlüssig, wie in dem Beispiel nach Fig. 2 und Fig. 3, absichern. Zusätzlich verhindern die Niederhalter 33 ein Öffnen der Rastgeometrien (Schnapphaken 16) an dem Lagertopf 17 bei Zugkräften Z.

Fig. 4 und Fig. 5 zeigen außerdem die Wälzlagereinheit 9, an welcher sowohl die Spindel 8 als auch eines der Antriebszahnräder 13 bereits angebracht sind. Diese Einbausituation ist gleichermaßen auf das Beispiel nach Fig. 2 und Fig. 3 übertragbar.

Die Wälzlagereinheit 9 nach dem vorstehend beschriebenen Beispiel nach Fig. 2 und Fig. 3 ist besonders einfach und kostengünstig und ist insbesondere im Falle von relativ geringen Belastungen von Vorteil. Wenn jedoch die Wälzlagereinheit 9 höheren Belastungen ausgesetzt wird, ist ggf. die Wälzlagereinheit 9 nach der vorstehend beschriebenen Ausführungsform vorteilhaft, da diese durch die Niederhalter 33 und Stützelemente 34 zusätzliche Sicherungen und Befestigungen hat, um höhere Lasten abzufangen, ohne dass dadurch die Anzahl der benötigten Bauteile, der Aufwand der (Vor-)Montage oder des Einbaus der erfindungsgemäßen Wälzlagereinheit 9 in die Spritzenpumpe 1 erhöht wird.

### Referenzzeichenliste

- 1: Medizinische Pumpe / Spritzenpumpe
- 2: Spritzengehäuse
- 3: Aufnahmefach
- 4: Spritze
- 5: Spritzenkolben
- 6: Antriebskopf
- 7: Antriebsrohr
- 8: Spindel
- 9: Wälzlagereinheit
- 10: Lagerplatte
- 11: Lagersitz
- 12: Getriebeabschnitt
- 13: Antriebszahnräder
- 14: Lagerbuchse
- 15: Umfangsnut
- 16: Schnapphaken / Vorspannelement
- 17: Lagertopf
- 18: Schlitze
- 19: Lagerkern
- 20: Flansch
- 21: Fase
- 22: Passungsabschnitt
- 23: erste Außenlauffläche
- 24: erste Wälzkörper
- 25: Auskragung
- 26: erste Innenlauffläche
- 27: zweite Innenlauffläche
- 28: zweite Wälzkörper
- 29: zweite Außenlauffläche
- 30: Spindelsitz
- 31: Fuß des Schnapphakens
- 32: Arme des Schnapphakens
- 33: Kopf des Schnapphakens / Niederhalter
- 34: Stützelement
- 35: Rückseitige Stirnfläche des Lagerkerns
- 36: Vorderseitige Stirnfläche des Lagerkerns
- 37: Montageschräge
- L: Längsachse der Wälzlagereinheit/ Lagerachse

## Patentansprüche

1. Doppelreihige Wälzlagereinheit (9) zur Lagerung beweglicher, rotierender Bauteile einer medizinischen Pumpe (1), vorzugsweise Spritzenpumpe, mit
einem Lagerkern (19), welcher eine erste, teilweise in eine Axialrichtung gewandte Innenlauffläche (26) für erste Wälzkörper (24) sowie eine zweite, der ersten Innenlauffläche (26) teilweise in Axialrichtung entgegengesetzt angeordnete Innenlauffläche (27) für zweite Wälzkörper (28) ausbildet;
einer Lagerbuchse (14), welche an einem Gehäuseabschnitt (10) anbringbar ist und eine der ersten Innenlauffläche (26) gegenüberliegende erste Außenlauffläche (23) für die ersten Wälzkörper (24) ausbildet;
einem Lagertopf (17), welcher eine der zweiten Innenlauffläche (27) gegenüberliegende zweite Außenlauffläche (29) für die zweiten Wälzkörper (28) ausbildet; und
zumindest einem Vorspannelement (16), welches den Lagertopf (17) mit der Lagerbuchse (14) mit definierter Vorspannung koppelt, um die handhabbar vormontierte, doppelreihige Wälzlagereinheit auszubilden, welches ein elastischer Abschnitt des Lagertopfs (17) oder der Lagerbuchse (14) ist, welcher entsprechend die Lagerbuchse (14) bzw. den Lagertopf (17) zumindest teilweise umgreift oder darin eingreift
**dadurch gekennzeichnet, dass** das zumindest eine Vorspannelement (16) einen oder mehrere Niederhalter (33) aufweist, welche dazu konfiguriert sind, sich radial nach außen hin an dem Gehäuseabschnitt (10) abstützen, um das zumindest eine Vorspannelement (16) in eine zugehörige Eingriffskontur (15) zu drücken, wenn die doppelreihige Wälzlagereinheit (9) in dem Gehäuseabschnitt (10) eingebaut ist, und
radial nach außen vorstehende Stützelemente (34) vorgesehen sind, welche zur Abstützung in einer zweiten axialen Lastrichtung an dem Gehäuseabschnitt (10) vorgesehen sind und welche elastisch sind, um radial nach innen einfedern zu können.

2. Doppelreihige Wälzlagereinheit (9) nach Anspruch 1, **dadurch gekennzeichnet, dass** die vorgespannte, doppelreihige Wälzlagereinheit (9) vorspannungseinstellfrei in der Pumpe (1) einbaubar ist.

3. Doppelreihige Wälzlagereinheit (9) nach Anspruch 1 oder 2, **dadurch gekennzeichnet, dass** der elastische Abschnitt des Lagertopfs (17) oder der Lagerbuchse (14) in Form von Schnapphaken bereitgestellt ist.

4. Doppelreihige Wälzlagereinheit (9) nach einem der vorstehenden Ansprüche, **dadurch gekennzeichnet, dass** die ersten Wälzkörper (24) und ersten Laufflächen (23, 26) mit den zweiten Wälzkörpern (28) und zweiten Laufflächen (27, 29) eine X-angeordnete Lagerung bilden.

5. Doppelreihige Wälzlagereinheit (9) nach einem der vorstehenden Ansprüche, **dadurch gekennzeichnet, dass** die ersten und/oder zweiten Wälzkörper (24, 28) eine Taumelbewegung des Lagerkerns (19) relativ zu dem Lagertopf (17) und der Lagerbuchse (14) ermöglichen.

6. Doppelreihige Wälzlagereinheit (9) nach einem der vorstehenden Ansprüche, **dadurch gekennzeichnet, dass** die Lagerbuchse (14) einen radial nach außen vorstehenden Flansch (20) ausbildet, welcher zur Abstützung in einer ersten axialen Lastrichtung an dem Gehäuseabschnitt (10) vorgesehen ist.

7. Doppelreihige Wälzlagereinheit (9) nach einem der vorstehenden Ansprüche, **dadurch gekennzeichnet, dass** der sich in Axialrichtung erstreckende hülsenförmige Abschnitt des Lagertopfs (17) die Stützelemente (34) ausbildet.

8. Doppelreihige Wälzlagereinheit (9) nach Anspruch 7, **dadurch gekennzeichnet, dass** die Stützelemente (34) rund um den Lagertopf (17) im Wechsel mit den Vorspannelementen (16), welche Schnapphaken bilden, in gleichen Abständen zueinander angeordnet sind.

9. Doppelreihige Wälzlagereinheit (9) nach Anspruch 8 **dadurch gekennzeichnet, dass** die Stützelemente (34) nach schräg radial außen abgeknickt sind, dass diese sich außen an der vorwärts gerichteten Fläche des Gehäuseabschnitts, welche eine Lagerplatte (10) ist, abstützen.

10. Medizinische Pumpe (1), vorzugsweise Spritzenpumpe, mit einer Spindel (8), vorzugsweise einer Zugspindel, welche durch eine doppelreihige Wälzlagereinheit (9) nach einem der vorstehenden Ansprüche drehbar und axialfest in einem Gehäuseabschnitt (10) der medizinischen Pumpe (1) gelagert ist.

## Claims

1. Double-row roller bearing unit (9) for supporting movable, rotating components of a medical pump (1), preferably a syringe pump, having
a bearing core (19) which forms a first inner bearing surface (26), partially facing in an axial direction, for first rolling elements (24) and a second inner bearing surface (27), partially arranged opposite the first inner bearing surface (26) in the axial direction, for second rolling elements (28);
a bearing sleeve (14) which can be attached to a housing portion (10) and forms a first outer bearing surface (23), opposite the first inner bearing surface (26), for the first rolling elements (24); and
a bearing pan (17) forming a second outer bearing surface (29), opposite the second inner bearing surface (27), for the second rolling elements (28); and
at least one pre-loading element (16) which couples the bearing pan (17) to the bearing sleeve (14) with a defined pre-load in order to form the handleably preassembled double-row roller bearing unit, wherein the pre-loading element (16) is an elastic portion of the bearing pan (17) or of the bearing sleeve (14) which correspondingly at least partially embraces or engages the bearing sleeve (14) or respectively the bearing pan (17),
**characterized in that** the at least one pre-loading element (16) comprises one or more hold-down devices (33) configured to rest against the housing portion (10) radially outwards in order to press the at least one pre-loading element (16) into an associated engagement contour (15) when the double-row roller bearing unit (9) is installed in the housing portion (10), and
support elements (34) projecting radially outwards are provided, which are provided for support at the housing portion (10) in a second axial load direction and which are elastic in order to be able to deflect radially inwards.

2. Double-row roller bearing unit (9) according to claim 1, **characterized in that** the pre-loaded double-row roller bearing unit (9) can be installed in the pump (1) without pre-load adjustment.

3. Double-row roller bearing unit (9) according to claim 1 or 2, **characterized in that** the elastic portion of the bearing pan (17) or of the bearing sleeve (14) is provided in the form of snap hooks.

4. Double-row roller bearing unit (9) according to one of the preceding claims, **characterized in that** the first rolling elements (24) and first bearing surfaces (23, 26) form an X-arranged bearing arrangement with the second rolling elements (28) and second bearing surfaces (27, 29).

5. Double-row roller bearing unit (9) according to one of the preceding claims, **characterized in that** the first and/or second rolling elements (24, 28) are rounded rolling elements and allow a wobbling movement of the bearing core (19) relative to the bearing pan (17) and the bearing sleeve (14).

6. Double-row roller bearing unit (9) according to one of the preceding claims, **characterized in that** the bearing sleeve (14) forms a flange (20) projecting radially outwards which is provided for support at the housing portion (10) in a first second axial load direction.

7. Double-row roller bearing unit (9) according to one of the preceding claims, **characterized in that** the sleeve-shaped portion of the bearing pan (17) that extends in the axial direction forms the support elements (34).

8. Double-row roller bearing unit (9) according to claim 7, **characterized in that** the support elements (34) are arranged alternatingly with the pre-loading elements (16), which form snap-fit hooks, around the bearing pan (17) at equal distances.

9. Double-row roller bearing unit (9) according to claim 8, **characterized in that** the support elements (34) are bent obliquely outwards in a radial direction, so that they are supported on the outside of the forward-facing surface of the housing portion (10), which is a bearing plate.

10. Medical pump (1), preferably a syringe pump, having a spindle (8), preferably a traction spindle, which is mounted rotatably and axially fixed in a housing portion (10) of the medical pump (1) by a double-row roller bearing unit (9) according to one of the preceding claims.

## Revendications

1. Unité de palier à rouleaux à double rangée (9) pour le montage de
composants rotatifs mobiles d'une pompe médicale (1), de préférence d'une pompe à seringue, avec un noyau de palier (19) qui forme une première surface de roulement intérieure (26) tournée partiellement dans une direction axiale pour de premiers corps de rouleaux (24) ainsi qu'une seconde surface de roulement intérieure (27) disposée partiellement à l'opposé de la première surface de roulement intérieure (26) en direction axiale pour de seconds corps de rouleaux (28) ;
une douille de palier (14) qui peut être appliquée sur une section de boîtier (10) et forme une première surface de roulement extérieure (23) opposée à la première surface de roulement intérieure (26) pour les premiers corps de rouleaux (24) ;
un pot de palier (17) qui forme une seconde surface de roulement extérieure (29) opposée à la seconde surface de roulement intérieure (27) pour les seconds corps de rouleaux (28) ; et
au moins un élément de précontrainte (16) qui couple le pot de palier (17) à la douille de palier (14) avec une précontrainte définie pour former l'unité de palier à rouleaux à double rangée prémontée de manière maniable, élément qui est une section élastique du pot de palier (17) ou de la douille de palier (14) qui entoure ou s'engage en conséquence au moins partiellement dans la douille de palier (14) ou le pot de palier (17)
**caractérisée en ce que** le au moins un élément de précontrainte (16) présente un ou plusieurs serre-flancs (33) qui sont configurés pour s'appuyer radialement vers l'extérieur sur la section de boîtier (10) pour pousser le au moins un élément de précontrainte (16) dans un contour d'engagement associé (15), lorsque l'unité de palier à rouleaux à double rangée (9) est montée dans la section de boîtier (10), et des éléments de support (34) faisant saillie radialement vers l'extérieur sont prévus, lesquels sont prévus pour appuyer dans une seconde direction de charge axiale sur la section de boîtier (10) et sont élastiques de façon à pouvoir se comprimer radialement vers l'intérieur.

2. Unité de palier à rouleaux à double rangée (9) selon la revendication 1, **caractérisée en ce que** l'unité de palier à rouleaux à double rangée (9) précontrainte peut être montée dans la pompe (1) sans réglage de précontrainte.

3. Unité de palier à rouleaux à double rangée (9) selon la revendication 1 ou 2, **caractérisée en ce que** la section élastique du pot de palier (17) ou de la douille de palier(14) est fournie sous forme de crochets d'encliquetage.

4. Unité de palier à rouleaux à double rangée (9) selon l'une quelconque des revendications précédentes, **caractérisée en ce que** les premiers corps de rouleaux (24) et les premières surfaces de roulement (23, 26) forment un palier disposé en X avec les seconds corps de rouleaux (28) et les secondes surfaces de roulement (27, 29).

5. Unité de palier à rouleaux à double rangée (9) selon l'une quelconque des revendications précédentes, **caractérisée en ce que** les premiers et/ou seconds corps de rouleaux (24, 28) permettent un mouvement de nutation du noyau de palier (19) par rapport au pot de palier (17) et à la douille de palier (14).

6. Unité de palier à rouleaux à double rangée (9) selon l'une quelconque des revendications précédentes, **caractérisée en ce que** la douille de palier (14) forme une bride (20) faisant saillie radialement vers l'extérieur qui est prévue pour appuyer dans une première direction de charge axiale sur la section de boîtier (10).

7. Unité de palier à rouleaux à double rangée (9) selon l'une quelconque des revendications précédentes, **caractérisée en ce que** la section en forme de manchon du pot de palier (17) s'étendant en direction axiale forme les éléments de support (34).

8. Unité de palier à rouleaux à double rangée (9) selon la revendication 7, **caractérisée en ce que** les éléments de support (34) sont disposés autour du pot de palier (17) en alternance avec les éléments de précontrainte (16) qui forment des crochets d'encliquetage à des distances égales les uns des autres.

9. Unité de palier à rouleaux à double rangée (9) selon la revendication 8, **caractérisée en ce que** les éléments de support (34) sont pliés en biais radialement vers l'extérieur, de sorte qu'ils appuient à l'extérieur sur la surface orientée vers l'avant de la section de boîtier qui est une plaque de palier (10).

10. Pompe médicale (1), de préférence pompe à seringue, avec une broche (8), de préférence une broche de traction, qui est montée de manière rotative et fixe axialement dans une section de boîtier (10) de la pompe médicale (1) par une unité de palier à rouleaux à double rangée (9) selon l'une quelconque des revendications précédentes.
